# EUROPÄISCHE PATENTSCHRIFT

(11) **EP 1 007 498 B1**
(45) Veröffentlichungstag und Bekanntmachung des Hinweises auf die Patenterteilung: **06.02.2002**
(21) Anmeldenummer: 98948831.7
(22) Anmeldetag: 13.08.1998
(51) Int. Cl.: C07C 49/697, C07C 45/46, C07C 245/20

(54) **VERFAHREN ZUR HERSTELLUNG VON 2-HALOGENINDAN-1-ONEN**
PROCESS FOR PREPARING 2-HALOGENATED INDAN-1-ONES
PROCEDE DE PREPARATION DE 2-HALOGENE INDANE-1-ONES

(30) Priorität: 25.08.1997 DE 19736922
(43) Veröffentlichungstag der Anmeldung: 14.06.2000
(73) Patentinhaber: Bayer Aktiengesellschaft, 51368 Leverkusen (DE)
(72) Erfinder: KOMOSCHINSKI, Joachim, D-51061 Köln (DE); FIEGE, Helmut, D-51373 Leverkusen (DE); STEFFAN, Guido, D-51519 Odenthal (DE); PAETZ, Klaus-Christian, D-51399 Burscheid (DE)
(86) Internationale Anmeldenummer: EP9805141
(87) Internationale Veröffentlichungsnummer: WO9910306

(56) Entgegenhaltungen:
- WO-A-96/20151
- DE-A- 2 640 358

## Beschreibung

Die vorliegende Erfindung betrifft ein verbessertes Verfahren zur Herstellung von 2-Halogenindan-1-onen.

Es ist bereits bekannt, daß man ausgehend von 1-Indan-1-onen in α-Position zur Ketogruppe halogenierte Indanone durch Halogenierung mit elementarem Chlor oder Brom herstellen kann. Hierzu ist es allerdings notwendig, zunächst das entsprechende Indanon-System aufzubauen. Dies ist schwierig, insbesondere, wenn sich im aromatischen Teil des Moleküls desaktivierende Substituenten wie beispielsweise NO₂, Cl, Br, Acetyl etc. befinden. Anschließend kann dann erst die Halogenierung durchgeführt werden (siehe z.B. J. Org. Chem. 24, 843 (1959) und DE-A 26 40 358). Diese Halogenierungen erfolgen nicht besonders selektiv, denn es werden auch mehrfach halogenierte Derivate gebildet, die dann in aufwendiger Weise abgetrennt werden müssen.

Indan-1-one selbst können mittels Friedel-Crafts-Reaktionen aus den entsprechenden Dihydrozimtsäuren hergestellt werden (siehe z.B. Organ. React., Band II, Kap. 4). Solche Zimtsäuren sind nicht leicht zugänglich, insbesondere, wenn sie z.B. Halogen- oder Trifluormethylsubstituenten enthalten. Nachteilig sind insbesondere die z.T. schlechte Zugänglichkeit der Aldehyd-Vorprodukte, die geringen Ausbeuten und der separate Halogenierungsschritt.

Eine Möglichkeit für die Herstellung von 5-Chlorindan-1-onen ist in Bull. Soc. Chim. de France (1973), 11, 3096 ausgehend von 3-Chlor-1-(4-chlorphenyl)-1-propanon beschrieben. Hier sind jedoch große Mengen einer Aluminiumtrichlorid/Natriumchlorid-Mischung und hohe Temperaturen von beispielsweise 180°C notwendig, um die Cyclisierung zu erreichen. Gemäß der WO 96/20151 wird das gleiche Ausgangsmaterial mit konzentrierter Schwefelsäure cyclisiert, wobei allerdings mit sehr stark verdünnten Lösungen gearbeitet werden muß, was eine Aufarbeitung erschwert und daher ziemlich unwirtschaftlich ist. Beide Verfahren liefern lediglich 5-Chlorindan-1-one, die dann, um 2-Halogenindan-1-one zu erhalten, in einer Folgestufe weiter halogeniert werden müssen.

Es wurde nun ein Verfahren zur Herstellung von 2-Halogenindan-1-onen der Formel 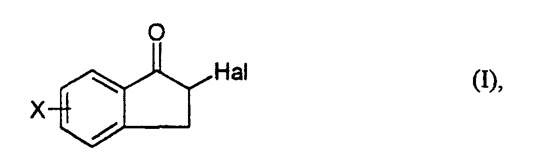 in der
- X: für Wasserstoff, Fluor, Chlor, Brom, Methyl, Trifluormethyl oder Methoxy und
- Hal: für Chlor oder Brom stehen,
gefunden, das dadurch gekennzeichnet ist, daß man ein Anilin der Formel 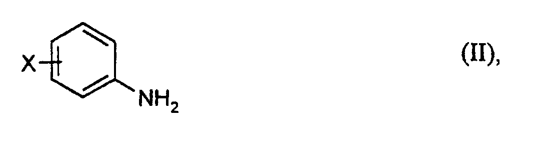 in der
- X: die bei Formel (I) angegebene Bedeutung hat,
in das entsprechende Diazoniumsalz der Formel (III) überführt 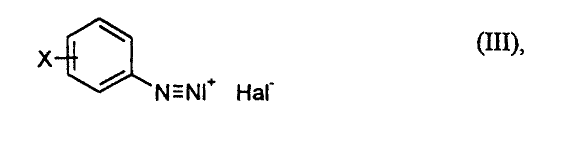 in der
- X und Hal: die bei Formel (I) angegebene Bedeutung haben,
dieses mit einer Acrylverbindung der Formel (IV) umsetzt 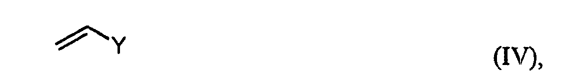 in der
- Y: für COR mit R = OH, Cl, Br, OC₁-C₄-Alkyl oder CN steht,
so eine Verbindung der Formel (V) erhält 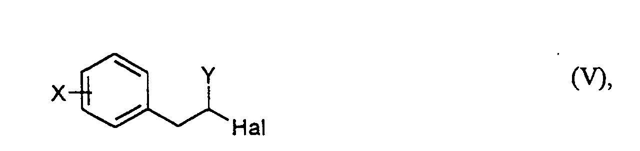 in der
- X und Hal: die bei Formel (I) und
- Y: die bei Formel (IV) angegebenen Bedeutungen haben,
dann Verbindungen der Formel (V) gegebenenfalls in Verbindungen der Formel (V)' überführt 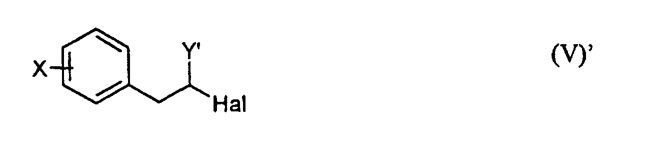 in der
- X und Hal: die bei Formel (I) angegebenen Bedeutungen haben und
- Y': für COOH, COCl oder COBr steht,
und eine Verbindung der Formel (V)' zu einem 2-Halogenindan-1-on der Formel (I) cyclisiert.

In den Formeln stehen vorzugsweise
- X: für Chlor, Brom oder Trifluormethyl,
- Hal: für Chlor,
- Y: für COOH, COOC₁-C₄-Alkyl oder CN und
- Y': für COOH oder COCl.

Die Umsetzung von Anilinen der Formel (II) zu Diazoniumsalzen der Formel (III) läßt sich entsprechend allgemein bekannten Methoden zur Herstellung von Diazoniumsalzen durchführen. Beispielsweise kann man das Anilin zusammen mit Salz- oder Bromwasserstoffsäure in Wasser lösen, dieser Lösung zunächst z.B. Natriumchlorid oder -bromid, weitere Salz- oder Bromwasserstoffsäure und Wasser zufügen und dann bei kontrollierter Temperatur (beispielsweise bei -5 bis +15°C) eine Lösung von Natriumnitrit in Wasser eindosieren. Man erhält so, gegebenenfalls nach Ablauf einer Nachrührzeit, eine wäßrige Lösung eines Diazoniumsalzes der Formel (III), die man so wie sie vorliegt mit einer Acrylverbindung der Formel (IV) umsetzen kann.

Die Umsetzung der Lösung des Diazoniumsalzes der Formel (III) mit einer Acrylverbindung der Formel (IV) kann man z.B. so durchführen, daß man die Acrylverbindung der Formel (IV) in einem mit Wasser mischbaren organischen Lösungsmittel, beispielsweise Aceton, löst und dieser Lösung die Lösung des Diazoniumsalzes hinzufügt (Vorsicht: Gasentwicklung!). Die Temperatur kann dabei z.B. 10 bis 60°C betragen. Es ist vorteilhaft einen Überschuß der Acrylverbindung einzusetzen, beispielsweise 105 bis 150 Gew.-% der theoretisch erforderlichen Menge. Weiterhin ist es vorteilhaft, die Umsetzung in Gegenwart von Kupfer(II)- und/oder Eisen(II)-salzen durchzuführen. Beispielsweise kann man dazu der Lösung der Acrylverbindung der Formel (IV) eine wäßrige Lösung von Kupfer(II)-chlorid oder -bromid oder Eisen(II)-chlorid hinzufügen. Pro 100 g Acrylverbindung der Formel (IV) kann man beispielsweise 2 bis 50 g Kupfer(II)- und/oder Eisen(II)-salze einsetzen. Vorzugsweise liegt diese Menge bei 3 bis 20 g.

Gegebenenfalls nach dem Ablauf einer Nachrührzeit kann man das Reaktionsgemisch beispielsweise aufarbeiten, indem man das Lösungsmittel und gegebenenfalls noch vorhandene überschüssige Acrylverbindung abdestilliert, den Rückstand mit Wasser versetzt und dann mit einem mit Wasser nicht mischbaren Lösungsmittel, etwa Diethylether, Chloroform oder Toluol versetzt. Nach dem Abtrennen der organischen Phase kann man daraus, gegebenenfalls nach deren Waschen mit Wasser, durch Abziehen des Lösungsmittels und eventuell noch vorhandener Acrylverbindung der Formel (IV) die hergestellte Verbindung der Formel (V) isolieren. Falls gewünscht kann sie noch weiter gereinigt werden, z.B. durch Kristallisation.

Wenn man eine Verbindung der Formel (V) vorliegen hat, bei der Y für COOC₁-C₆-Alkyl oder CN steht ist nunmehr zunächst eine Überführung in eine Verbindung der Formel (V)' durchzuführen, in der Y ' für COOH, COCI oder COBr steht. Beispielsweise kann man durch eine an sich bekannte Esterverseifung aus Verbindungen der Formel (V) Verbindungen der Formel (V)' mit Y' = COOH erhalten. Die letzteren kann man gewünschtenfalls in Verbindungen der Formel (V)' mit Y' = COCI oder COBr überführen, wenn man z.B. eine an sich bekannte Säurehalogenidherstellung anwendet, beispielsweise eine Umsetzung einer Verbindung der Formel (V) oder (V)' mit Y bzw. Y' = COOH mit Thionylchlorid, Phosphortrichlorid oder Phosphortribromid durchführt.

Für den erfindungsgemäßen Cyclisierungsschritt einer Verbindung der Formel (V)' zu einem 2-Halogenindan-1-on sind zwei Varianten anwendbar.

### 1. Variante

Eine Verbindung der Formel (V)' mit Y' = COOH wird mit einem Kondensationsmittel versetzt und erhitzt. Als Kondensationsmittel kommen beispielsweise Polyphosphorsäure, Mischungen aus Polyphosphorsäure und Phosphorpentoxid, Methansulfonsäure, Mischungen aus Methansulfonsäure und Phosphorpentoxid, Schwefelsäure, Flußsäure oder Trifluormethansulfonsäure in Frage. Das Kondensationsmittel wird im allgemeinen im Überschuß eingesetzt, beispielsweise auf 100 g der Verbindung der Formel (V)' 500 bis 5000 g, vorzugsweise 600 bis 4000 g Kondensationsmittel. Die Reaktionstemperatur kann beispielsweise 25 bis 120°C, vorzugsweise 30 bis 110°C betragen. Aus dem nach der Kondensationsreaktion vorliegenden Reaktionsgemisch kann man das hergestellte 2-Halogenindan-1-on beispielsweise gewinnen, indem man das Reaktionsgemisch abkühlt, in Eiswasser schüttet, die organischen Bestandteile mit einem mit Wasser nicht mischbaren Lösungsmittel, z.B. Diethylether, Chloroform oder Toluol aufnimmt, die organische Phase trocknet und das Lösungsmittel entfernt.

### 2. Variante

Eine Verbindung der Formel (V)' mit Y' = COCl oder COBr wird mit einem Friedel-Crafts-Katalysator versetzt und erhitzt. Als Friedel-Crafts-Katalysatoren kommen z.B. Aluminiumtrichlorid, Eisentrichlorid, Zinkchlorid, Aluminiumbromid, Mischungen davon und Schmelzen aus Aluminiumtrichlorid/Natriumchlorid in Frage. Die Friedel-Crafts-Katalysatoren kann man z.B. suspendiert in einem inerten Lösungsmittel und in Mengen von z.B. 0,9 bis 1,3 Mol, bezogen auf ein Mol der Verbindung der Formel (V)' mit Y' = COCI oder COBr einsetzen. Geeignete Reaktionstemperaturen sind beispielsweise solche von 50 bis 100°C. Die Aufarbeitung des Reaktionsgemisches kann wie bei der 1. Variante erfolgen.

Mit dem erfindungsgemäßen Verfahren erhält man selektiv und in guten Ausbeuten in 2-Position halogenierte Indan-1-one. Diese können gegebenenfalls nach weiteren Funktionalisierungen als Ausgangsmaterial für Anwendungen verwendet werden, wie sie beispielsweise in der WO 95/29171 und der DE-A 26 40 358 genannt sind.

Es ist überraschend, daß die Umkehrung des bekannten Syntheseweges für 2-Halogenindan-1-one, d.h. die erfindungsgemäße Synthese des Indanon-Gerüstes als letzte Verfahrensstufe durchzuführen, soviel günstiger ist als die Halogenierung von Indanonen, zumal die Synthese des Indanon-Gerüstes als letzte Verfahrensstufe bei der Herstellung von 5-Halogenindan-1-onen nicht besonders vorteilhaft ist.

### Beispiele

### Beispiel 1

129 g 3-Chloranilin wurden in einer Lösung von 480 ml Wasser und 108 ml konzentrierter wäßriger Salzsäure in der Wärme gelöst und danach auf Raumtemperatur gekühlt (→ Lösung I). Dann wurden 68,4 g Natriumnitrit in 104 ml Wasser gelöst (→ Lösung II). Zur Lösung I wurden nun 103 g Natriumchlorid, 140 ml konzentrierte wäßrige Salzsäure und 108 ml Wasser gegeben und die Lösung auf 0°C abgekühlt. Anschließend wurde die Lösung II so unter die Oberfläche der Lösung I getropft, daß die Temperatur nicht über +5°C anstieg. Nach beendeter Zugabe wurde noch 30 Minuten weitergerührt.

In einem weiteren Reaktionsgefäß wurden 1600 ml Aceton und 387 g Acrylsäure vermischt, auf 60°C erwärmt und hierzu eine Lösung von 20 g Kupfer(II)-chlorid in 50 ml Wasser gegeben.

Innerhalb von 1 Stunde wurden die vereinigten Lösungen I + II zu der 55 bis 60°C warmen acetonischen Acrylsäurelösung zugefügt (Gasentwicklung!). Nach beendeter Zugabe wurde noch 30 Minuten unter Rückfluß erhitzt und anschließend das Aceton und überschüssige Acrylsäure abdestilliert. Zu der verbleibenden Lösung wurden 560 ml Wasser und 700 ml Toluol gegeben, die organische Phase abgetrennt, diese mit 2x 600 ml Wasser gewaschen und dann das Toluol und restliche Acrylsäure daraus abdestilliert.

Auf diese Weise wurden 162,4 g 2-Chlor-3-(3-chlorphenyl)-propionsäure erhalten. Das Produkt kann, falls gewünscht, durch Kristallisation weiter gereinigt werden.

### Beispiel 2

Es wurde verfahren wie in Beispiel 1 beschrieben, jedoch wurde anstelle von 3-Chloranilin 4-Chloranilin eingesetzt. Es wurde 2-Chlor-3-(4-chlorphenyl)-propionsäure in einer Ausbeute von 74 % erhalten.

### Beispiel 3

11 g 2-Chlor-3-(4-chlorphenyl)-propionsäure wurden in 140 g Polyphosphorsäure aufgenommen und 90 Minuten bei 100°C gerührt. Danach wurde das Reaktionsgemisch auf 55°C abgekühlt und die Masse in 500 g Eiswasser eingetragen. Der organische Anteil wurde mit Chloroform extrahiert, anschließend die organische Phase getrocknet und das Chloroform abdestilliert. Man erhielt so 9,5 g 2,6-Dichlorindan-1-on.

### Beispiel 4

Es wurde verfahren wie in Beispiel 3 beschrieben, jedoch wurde als Ausgangsmaterial in die Cyclisierung 2-Chlor-3-(3-chlorphenyl)-propionsäure eingesetzt. Hierbei erhielt man eine Gesamtausbeute an Dichlorindan-1-onen von 78 %. Der Gehalt an 2,5-Dichlorindan-1-on betrug 75 Gew.-%, der Gehalt an 2,7-Dichlorindan-1-on 25 Gew.-%.

### Beispiel 5

219 g 2-Chlor-3-(4-chlorphenyl)-propionsäure wurden vorgelegt und auf 70°C erhitzt. Nun tropfte man 226 g Thionylchlorid zu (Gasentwicklung!) und hielt die Mischung solange am Rückfluß, bis die Gasentwicklung beendet war. Anschließend wurde zunächst das überschüssige Thionylchlorid und dann im Vakuum das 2-Chlor-3-(4-chlorphenyl)-propionsäurechlorid (Kp bei 7 bis 8 mbar 132 bis 135°C) abdestilliert (226 g Produkt).

Das auf diese Weise erhaltene Säurechlorid wurde anschließend cyclisiert. Hierzu suspendierte man 6,7 g wasserfreies Aluminiumchlorid in 75 ml einer bei 60 bis 95°C siedenden Benzinfraktion, brachte diese Mischung auf 60°C und tropfte eine Lösung von 11,9 g des Säurechlorids in 50 ml des genannten Benzins dazu. Anschließend wurde 2 Stunden bei 60°C nachgerührt. Die Reaktionsmischung wurde auf Eis gegossen, dem wenig Salzsäure zugefügt war. Anschließend extrahierte man den organischen Anteil mit Diethylether, destillierte den Ether ab und erhielt das gewünschte 2,6-Dichlorindan-1-on (9,8 g).

### Beispiel 6

Es wurde verfahren wie in Beispiel 5 beschrieben, jedoch wurde anstelle von 2-Chlor-3-(4-chlorphenyl)-propionsäure jetzt 2-Chlor-3-(3-chlorphenyl)-propionsäure eingesetzt. Man erhielt in einer Gesamtausbeute von 96,4 % Dichlorindan-1-one. Der Gehalt an 2,5-Dichlorindan-1-on betrug 85 Gew.-%, der Gehalt an 2,7-Dichlorindan-1-on 15 Gew.-%.

## Patentansprüche

1. Verfahren zur Herstellung von 2-Halogenindan-1-onen der Formel 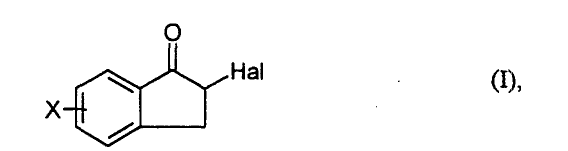 in der
X für Wasserstoff, Fluor, Chlor, Brom, Methyl, Trifluormethyl oder Methoxy und
Hal für Chlor oder Brom stehen,
**dadurch gekennzeichnet, daß** man ein Anilin der Formel 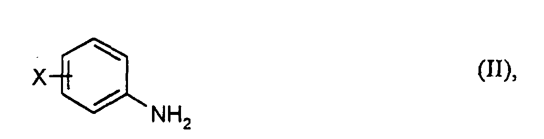 in der
X die bei Formel (I) angegebene Bedeutung hat,
in das entsprechende Diazoniumsalz der Formel (III) überführt 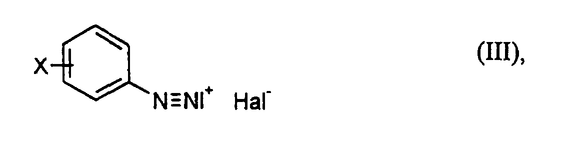 in der
X und Hal die bei Formel (1) angegebene Bedeutung haben,
dieses mit einer Acrylverbindung der Formel (IV) umsetzt 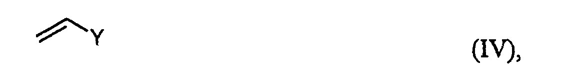 in der
Y für COR mit R = OH, Cl, Br, OC₁-C₄-Alkyl oder CN steht,
so eine Verbindung der Formel (V) erhält 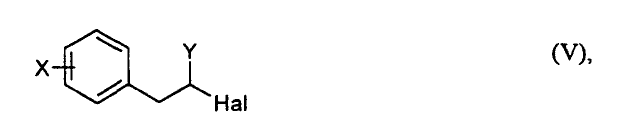 in der
X und Hal die bei Formel (I) und
Y die bei Formel (IV) angegebenen Bedeutungen haben,
dann Verbindungen der Formel (V) gegebenenfalls in Verbindungen der Formel (V)' überführt 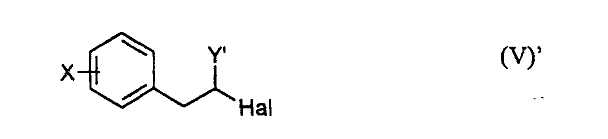 in der
X und Hal die bei Formel (I) angegebenen Bedeutungen haben und
Y' für COOH, COCI oder COBr steht,
und eine Verbindung der Formel (V)' zu einem 2-Halogenindan-1-on der Formel (I) cyclisiert.

2. Verfahren nach Anspruch 1, **dadurch gekennzeichnet, daß** man die Umsetzung des Diazoniumsalzes der Formel (III) mit einer Acrylverbindung der Formel (IV) so vornimmt, daß man die Acrylverbindung der Formel (IV) in einem mit Wasser mischbaren organischen Lösungsmittel vorlegt und zu dieser Lösung die Lösung des Diazoniumsalzes hinzufügt, die Reaktionstemperatur bei 10 bis 60°C hält und einen Überschuß an der Acrylverbindung einsetzt.

3. Verfahren nach Ansprüchen 1 und 2, **dadurch gekennzeichnet, daß** man die Umsetzung des Diazoniumsalzes der Formel (III) mit einer Acrylverbindung der Formel (IV) in Gegenwart von Kupfer(II)- und/oder Eisen(II)-salzen durchführt.

4. Verfahren nach Ansprüchen 1 bis 3, **dadurch gekennzeichnet, daß** man den Cyclisierungsschritt beim Einsatz einer Verbindung der Formel (V)' mit Y' = COOH durch Versetzen mit einem Kondensationsmittel und bei einer Temperatur von 25 bis 120°C durchführt.

5. Verfahren nach Ansprüchen 1 bis 3, **dadurch gekennzeichnet, daß** man den Cyclisierungsschritt beim Einsatz einer Verbindung der Formel (V)' mit Y' = COCI oder COBr durch Zugabe eines Friedel-Crafts-Katalysators und einer Reaktionstemperatur von 50 bis 100°C durchführt.

## Claims

1. Process for the production of 2-halo-1-indanones of the formula 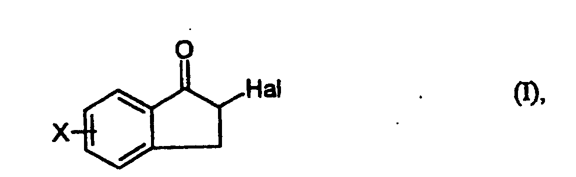 in which
X denotes hydrogen, fluorine, chlorine, bromine, methyl, trifluoromethyl or methoxy and
Hal denotes chlorine or bromine,
**characterised in that** an aniline of the formula 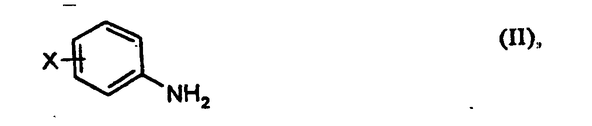 in which
X has the meaning stated in formula (I),
is converted into the corresponding diazonium salt of the formula (III) 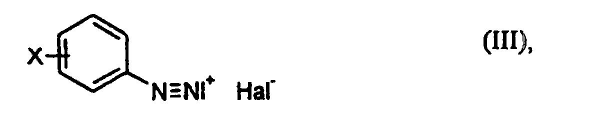 in which
X and Hal have the meaning stated in formula (I),
this latter is reacted with an acrylic compound of the formula (IV) 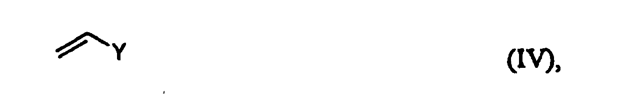 in which
Y denotes COR where R = OH, Cl, Br, OC₁-C₄-alkyl or CN,
a compound of the formula (V) is thus obtained 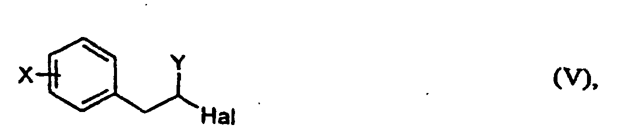 in which
X and Hal have the meaning stated in formula (I) and
Y has the meaning stated in formula (IV),
then compounds of the formula (V) are optionally converted into compounds of the formula (V)' 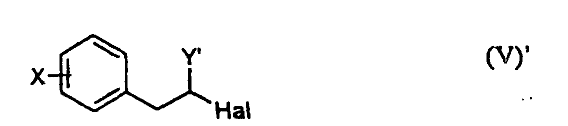 in which
X and Hal have the meanings stated in formula (I) and
Y' denotes COOH, COCl or COBr,
and a compound of the formula (V)' is cyclised to yield a 2-halo-1-indanone of the formula (I).

2. Process according to claim 1, **characterised in that** the reaction of the diazonium salt of the formula (III) with an acrylic compound of the formula (IV) is performed in such a manner that the acrylic compound of the formula (IV) is initially introduced into a water-miscible organic solvent and the solution of the diazonium salt is added to this solution, the reaction temperature is maintained at 10 to 60°C and an excess of the acrylic compound is used.

3. Process according to claims 1 and 2, **characterised in that** the reaction of the diazonium salt of the formula (III) with an acrylic compound of the formula (IV) is performed in the presence of copper(II) and/or iron(II) salts.

4. Process according to claims 1 to 3, **characterised in that**, when a compound of the formula (V)' where Y' = COOH is used, the cyclisation step is performed by combination with a condensing agent and at a temperature of 25 to 120°C.

5. Process according to claims 1 to 3, **characterised in that**, when a compound of the formula (V)' where Y' = COCl or COBr is used, the cyclisation step is performed by addition of a Friedel-Crafts catalyst and at a temperature of 50 to 100°C.

## Revendications

1. Procédé pour la préparation de 2-halogénoindane-1-ones de formule 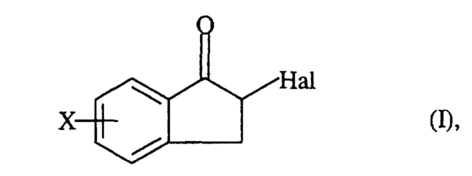 dans laquelle
X représente l'hydrogène, le fluor, le chlore, le brome, un groupe méthyle, trifluorométhyle ou méthoxy et
Hal représente le chlore ou le brome,
**caractérisé en ce que** l'on convertit une aniline de formule 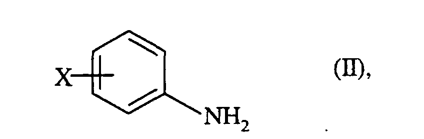 dans laquelle
X a la signification indiquée en référence à la formule (I),
en le sel de diazonium correspondant de formule (III) 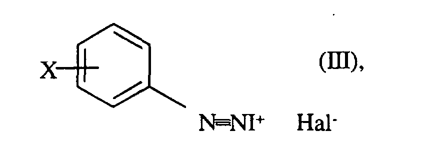 dans laquelle
X et Hal ont les significations indiquées en référence à la formule (I),
qu'on fait réagir avec un dérivé acrylique de formule (IV) 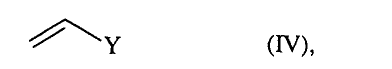 dans laquelle
Y représente COR avec R = OH, Cl, Br, O-alkyle en C₁-C₄ ou CN,
ce qui donne un composé de formule (V) 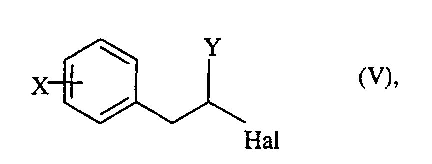 dans laquelle
X et Hal ont les significations indiquées en référence à la formule (I) et
Y a les significations indiquées en référence à la formule (IV),
on convertit ensuite le cas échéant les composés de formule (V) en composés de formule (V)' 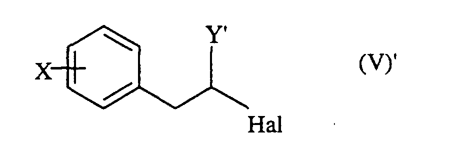 dans laquelle
X et Hal ont les significations indiquées en référence à la formule (I) et
Y' représente COOH, COCl ou COBr,
et on cyclise un composé de formule (V)' en une 2-halogénoindane-1-one de formule (I).

2. Procédé selon revendication 1, **caractérisé en ce que** la réaction entre le sel de diazonium de formule (III) et un dérivé acrylique de formule (IV) est réalisée par introduction du dérivé acrylique de formule (IV) dans un solvant organique miscible à l'eau et addition à cette solution de la solution du sel de diazonium, en maintenant une température de réaction de 10 à 60°C et en utilisant le dérivé acrylique en excès.

3. Procédé selon les revendications 1 et 2, **caractérisé en ce que** la réaction entre le sel de diazonium de formule (III) et un dérivé acrylique de formule (IV) est réalisée en présence de sels cuivriques et/ou de sels ferreux.

4. Procédé selon les revendications 1 à 3, **caractérisé en ce que** la cyclisation, au départ d'un composé de formule (V)' dans laquelle Y' = COOH, est réalisée par addition d'un agent de condensation et à une température de 25 à 120°C.

5. Procédé selon les revendications 1 à 3, **caractérisé en ce que** la cyclisation, au départ d'un composé de formule (V)' dans laquelle Y' = COCl ou COBr, et réalisée par addition d'un catalyseur de Friedel-Crafts et à une température de réaction de 50 à 100°C.
